# EUROPÄISCHE PATENTSCHRIFT

(11) **EP 1 165 525 B1**
(45) Veröffentlichungstag und Bekanntmachung des Hinweises auf die Patenterteilung: **03.08.2005**
(21) Anmeldenummer: 00914118.5
(22) Anmeldetag: 08.03.2000
(51) Int. Cl.: C07D 251/62

(54) **VERFAHREN ZUR HERSTELLUNG VON FESTEM MELAMIN**
METHOD FOR PRODUCING SOLID MELAMINE
PROCEDE DE PRODUCTION DE MELAMINE SOLIDE

(30) Priorität: 15.03.1999 AT 45099; 15.03.1999 AT 45199
(43) Veröffentlichungstag der Anmeldung: 02.01.2002
(73) Patentinhaber: AMI Agrolinz Melamine International GmbH, 4020 Linz (AT)
(72) Erfinder: COUFAL, Gerhard, A-4060 Leonding (AT)
(74) Vertreter: Gross, Felix, Dr.
(86) Internationale Anmeldenummer: PCT/EP2000/002012
(87) Internationale Veröffentlichungsnummer: WO 2000/055142

(56) Entgegenhaltungen:
- EP-A- 0 808 836
- WO-A-97/20826
- WO-A-97/47609

## Beschreibung

Die Erfindung betrifft ein Verfahren zur Herstellung von festem Melamin, bei dem eine Dispersion von Ammoniak und flüssigem Melamin entspannt wird, wobei sich das Melamin in fester Form abscheidet.

Melamin wird bevorzugt durch Pyrolyse von Harnstoff hergestellt, wobei sowohl Niederdruckverfahren als auch Hochdruckverfahren, wie sie beispielsweise in "Ullmann's Encyclopedia of Industrial Chemistry, Vol A 16, 5^{th} ed (1990), Seiten 171-185" beschrieben sind, zur Anwendung kommen können. Das bei der Melaminsynthese anfallende Melamin enthält je nach Herstellverfahren etwa 94-98 Gew.% Melamin, sowie insbesondere Melam, Melem, Ureidomelamin, Ammelin und Ammelide als wesentliche Verunreinigungen und muß für anspruchsvollere Anwendungsgebiete durch besondere Verfahrensschritte weiter gereinigt werden.
Um Melamin in fester Form zu erhalten, kann die flüssige Melaminschmeize beispielsweise mit Wasser, mit wäßrigen melaminhaltigen Lösungen oder Suspensionen, mit kalten festen Inertstoffen oder festem Melamin. gemäß AT 159/98, beispielsweise in einem Wirbelbett, abgekühlt werden. Besonders vorteilhaft ist es, eine ammoniakhältige Melaminschmelze beispielsweise gemäß WO97/20826 in einen Abkühlbehälter einzusprühen und zu entspannen, in dem eine Ammoniakatmosphäre vorliegt, wobei sich festes Melamin in reiner Form abscheidet. Allerdings können bei diesem Verfahren nicht bei allen Druck- und Temperaturbedingungen optimale Ergebnisse erreicht werden.

Es stellte sich demnach die Aufgabe, ein Verfahren zu finden, bei dem unabhängig von der jeweiligen Temperatur und vom jeweiligen Druck, festes Melamin in guter Qualität durch Entspannen auch in einem breiten Temperatur- und Druckbereich, insbesondere auch bei niedrigen Drücken der Schmelze, erhalten werden kann.

Es wurde nun gefunden, daß diese Aufgabe dadurch gelöst werden kann, daß die zu entspannende Melaminschmelze zusätzlich zum gelösten Ammoniak noch überschüssiges Ammoniak enthält, sodaß eine zweiphasige Mischung als Dispersion von Ammoniak und flüssigem Melamin vorliegt.

Gegenstand der Erfindung ist demnach ein Verfahren zur Herstellung von festem Melamin durch Entspannen von flüssigem, ammoniakhältigem Melamin, dadurch gekennzeichnet, daß
a) das flüssige, ammoniakhältige Melamin mit überschüssigem Ammoniak vermischt wird, wobei sich eine Dispersion von gasförmigen Ammoniak und flüssigem Melamin bildet,
b) die Dispersion gegebenenfalls unter Ammoniakdruck verweilen gelassen wird,
c) die Dispersion entspannt wird, wobei sich Melamin in fester Form abscheidet;
d) das feste Melamin gegebenenfalls unter Ammoniakdruck verweilen gelassen wird,
e) worauf in beliebiger Reihenfolge gegebenenfalls auf Atmosphärendruck weiterentspannt, auf Raumtemperatur abgekühlt und das Melamin isoliert wird.

Das Ammoniak (Gasphase) ist bevorzugt überkritisch und in fein verteilter Form in der flüssigen Meiarninschmelze (Flüssigphase) dispergiert, wobei ein sehr feinverteilter "Melaminschaum" entsteht. Beim Vermischen bildet sich eine Dispersion von Melamin und Ammoniak, wobei sich das flüssige Melamin mit Ammoniak sättigt. Die flüssige Melaminphase ist bevorzugt mit Ammoniak gesättigt. Erfindungsgemäß ist es sowohl möglich, daß Ammoniak im flüssigen Melamin dispergiert ist, als auch, daß flüssiges Melamin im Ammoniak dispergiert ist. Wesentlich ist, daß die Gesamtmenge des Ammoniaks (gelöstes Ammoniak und in der Gasphase befindliches Ammoniak) so groß ist, daß bei der Entspannung soviel Wärme abgeführt wird, wie zur Verfestigung des Meiamins notwendig―ist. Ein besonderer Vorteil der Erfindung liegt demnach insbesondere darin, daß auch bei geringeren Drücken und höheren Temperaturen der Melaminschmelzen, bei denen relativ wenig Ammoniak in der Melaminschmelze gelöst ist, mit Hilfe des überschüssigen, in der Schmelze dispergierten Ammoniaks bei der Entspannung eine zur Verfestigung des Melamins ausreichende Wärmeabfuhr möglich ist. Die Menge des überschüssigen Ammoniaks in der Melaminschmelze richtet sich insbesondere danach, wie hoch Temperatur, Druck und Ammoniaksättigung der Schmelze vor der Entspannung sind und wie weit unterhalb seines Schmelzpunktes das bereits verfestigte Melamin weiter gekühlt werden soll. Bei hohen Temperaturen und niedrigen Drücken der Schmelze sind demnach höhere Mengen an überschüssigem Ammoniak nötig, als bei Temperaturen, die knapp über dem vom jeweiligen Ammoniakdruck abhängigen Schmelzpunkt des Melamins liegen. Umgekehrt sind bei hohen Drücken geringere Mengen an überschüssigem Ammoniak nötig. Die Menge an überschüssigem Ammoniak kann demnach in weiten Grenzen variieren.

Da der Schmelzpunkt des Melamins bei niedrigeren Drücken nach dem Entspannen höher liegt als bei hohen Drücken, ist es erfindungsgemäß auch möglich ― insbesondere wenn die Schmelzetemperatur nicht allzu hoch über dem jeweiligen druckabhängigen Schmelzpunkt liegt und die Menge an überschüssigem Ammoniak nicht sehr groß ist - daß die Temperatur bei der Verfestigung gleich bleibt, bzw. sogar steigt.

Es ist gemäß Erfindung bevorzugt, flüssiges ammoniakhältiges Melamin bei Drücken von etwa 50 bar bis etwa 1000 bar mit Ammoniak zu vermischen und anschließend auf einen Druck von etwa 1 bis 200 bar zu entspannen, wobei sich Melamin in fester Form abscheidet. Der Druck sowohl vor als auch nach dem Entspannen kann je nach gewählter Verfahrensweise in einem großen Bereich schwanken. Bevorzugt liegt die Obergrenze des Druckes vor dem Entspannen bei etwa 600 bar, besonders bevorzugt bei etwa 350 bar oder bei etwa 250 bar. Sie kann aber auch bei etwa 150 bar oder bei etwa 130 bar liegen. Die Untergrenze des Druckes vor dem Entspannen liegt bevorzugt bei etwa 60 bis 80 bar. Der Druck nach dem Entspannen kann ebenfalls in einem großen Bereich schwanken. Für den Fall, daß ein Tempern unmittelbar angeschlossen wird, wird auf höhere Drücke entspannt, im anderen Fall ist ein Entspannen bis auf Atmosphärendruck möglich. Der Druck nach dem Entspannen liegt demnach bevorzugt bei etwa 1 bis 100 oder 150 bar, besonders bevorzugt bei etwa 1 bis 60 bar. Er kann aber auch bei etwa 10 bis 20 bar liegen.

Die Temperatur des flüssigen ammoniakhältigen Melamins liegt beim Mischen mit Ammoniak bzw. vor dem Entspannen bevorzugt in einem Bereich von etwa 60 °C über dem vom jeweiligen Ammoniakdruck abhängigen Schmelzpunkt des Melamins bis knapp über dem vom jeweiligen Ammoniakdruck abhängigen Schmelzpunkt des Melamins, besonders bevorzugt bei Temperaturen, die zwischen etwa 1 und 40 °C, ganz besonders bevorzugt zwischen etwa 1 und 20 °C über dem vom jeweiligen Ammoniakdruck abhängigen Schmelzpunkt des Melamins liegen. Am günstigsten ist es, wenn die Temperatur möglichst knapp über dem vom jeweiligen Ammoniakdruck abhängigen Schmelzpunkt des Melamins liegt. Insbesondere bevorzugt liegt die gewünschte Entspannungstemperatur bei unter etwa 350 °C. Bevorzugt ist das flüssige, ammoniakhältige Melamin mit Ammoniak gesättigt.

Das Vermischen des flüssigen ammoniakhältigen Melamins mit überschüssigem Ammoniak unter Bildung einer Dispersion kann in geeigneten Mischeinrichtungen erfolgen, beispielsweise mit Hilfe von Mischern, Rührern, in Reaktoren mit selbstansaugenden Rührern, mit Hilfe von Statikmischern, Injektoren, Ejektoren oder anderen geeigneten Mischeinrichtungen. Die Melaminschmelze kann sowohl mit gasförmigem als auch mit flüssigem Ammoniak vermischt werden, wobei sich die Schmelze jedoch auch im Falle der Verwendung von flüssigem Ammoniak nicht verfestigen darf. Bevorzugt wird dabei die Temperatur der Schmelze erniedrigt und dabei in die Nähe der gewünschten bzw. auf die gewünschte Temperatur gebracht.

Vor der Entspannung können sowohl Druck als auch Temperatur gegebenenfalls in beliebiger Weise, sowohl vor als auch nach dem Mischen von Melamin mit Ammoniak, erhöht, erniedrigt oder konstant gehalten werden, wobei sich die Schmelze jedoch nicht verfestigen darf.
Zur Erzielung einer besonders guten Melaminqualität ist es vorteilhaft, die flüssige Melaminschmelze vor dem Entspannen entweder vor oder nach dem Mischen des flüssigen Melamins und Ammoniaks, unter Ammoniakdruck verweilen zu lassen (Aging). Dies geschieht bevorzugt während etwa 1 min bis 10 h in Abhängigkeit von den gewählten Verfahrensbedingungen in einem Temperaturbereich von etwa 350 °C bis knapp über dem vom jeweiligen Ammoniakdruck abhängigen Schmelzpunkt des Melamins, bevorzugt bei Temperaturen, die etwa 1 bis 60 °C, besonders bevorzugt bei etwa 1 bis 40 °C, weiters besonders bevorzugt bei etwa 1 bis 20 °C über dem vom jeweiligen Ammoniakdruck abhängigen Schmelzpunkt des Melamins liegen. Dabei ist es vorteilhaft, die Temperatur des flüssigen Melamins abzusenken, beispielsweise durch Zufuhr von flüssigem oder gasförmigem Ammoniak. Der Druck beim Aging liegt dabei im Bereich von etwa 50 bis 1000 bar, bevorzugt bei etwa 80 bis 600 bar, besonders bevorzugt bei etwa 130 bis 400 bar. Für den Fall, daß das Aging nach dem Vermischen von Melamin und Ammoniak erfolgt, ist darauf zu achten, daß die Dispersion während des Agings erhalten bleibt.

Die Dispersion von Ammoniak und flüssigem Melamin wird bevorzugt bei einer Temperatur, die etwa 1 bis 60 °C, besonders bevorzugt bei etwa 1 bis 40 °C, weiters besonders bevorzugt bei etwa 1 bis 20 °C über dem vom jeweiligen Ammoniakdruck abhängigen Schmelzpunkt des Melamins liegt, entspannt, wobei sich das Melamin unter seinem nun bei niedrigerem Druck höher liegenden Schmelzpunkt in fester Form abscheidet.

Gemäß einer ersten Ausführungsform der Erfindung wird die Melamin-Ammoniak-Dispersion anschließend an das Vermischen entspannt. Dabei kann auch zusätzlich Ammoniak zugeführt werden. Bevorzugt ist es, die Dispersion in einen separaten, eine Ammoniakatmosphäre enthaltenden, gegebenenfalls beheizten Behälter zu entspannen. Die Melamindispersion kann dabei beispielsweise über Düsen, z.B. Einstoffdüsen, Zweistoffdüsen oder Venturidüsen, über injektoren oder Ejektoren in den Behälter gesprüht werden.

Gemäß einer weiteren Ausführungsform der Erfindung ist es auch möglich, das flüssige Melamin oder eine Dispersion von Melamin und Ammoniak mit dem überschüssigen Ammoniak beim Entspannen zu mischen. Dies gelingt bevorzugt in speziellen Misch- und Förderapparaturen, beispielsweise in Injektoren oder Ejektoren, in denen sich Ammoniak als Treibgas mit dem flüssigen Melamin oder flüssiges Melamin als Treibmedium mit Ammoniak vermischt und die sich dabei bildende Dispersion gleichzeitig unter Druckabfall in den Entspannungsbehälter gefördert wird.

Es ist auch möglich, das feste Melamin im Anschluß an die Entspannung umzuwälzen, beispielsweise durch Rühren oder durch Entspannen der Melaminschmelze unter Verfestigung beispielsweise in eine rotierende Trommel oder in ein Fließbett.

Durch das Entspannen kann sich die Temperatur des Melamins sowohl erniedrigen als auch erhöhen oder sie kann auch gleich bleiben. Die Temperatur des festen Melamins nach dem Entspannen liegt vor allem bei höherem Ammoniaküberschuß zumeist niedriger als die Temperatur der Dispersion vor dem Entspannen. Erfindungsgemäß ist es jedoch aufgrund des höheren Melamin-Schmelzpunktes bei niedrigeren Drücken auch möglich und besonders vorteilhaft, daß die Temperatur des festen Melamins nach dem Entspannen aufgrund der freiwerdenden Kristallisationswärme gleich bleibt oder sogar ansteigt.

Es erweist sich ebenfalls als vorteilhaft, das Melamin auch nach der Verfestigung unter Ammoniakdruck verweilen zu lassen. Dabei wird das feste Melamin nach dem Entspannen beispielsweise während etwa 10 sec bis 20 h, bevorzugt während etwa 1 min bis 2 h in einem Temperaturbereich, der zwischen etwa 150 °C und dem vom jeweiligen Ammoniakdruck abhängigen Schmelzpunkt des Melamins liegt, in einer Ammoniakatmosphäre, gegebenenfalls unter mechanischem Rühren oder in einer rotierenden Trommel oder durch pneumatische Umwälzung, beispielsweise in einem Fließbett verweilen gelassen. Die Verweilzeit beim Tempern kann dabei umso kürzer sein, je höher Temperatur und Druck sind. Bevorzugt liegt diese Temperatur möglichst knapp, vorteilhafterweise bis zu 10 °C, besonders bevorzugt bis zu 5 °C unterhalb des vom jeweiligen Ammoniakdruck abhängigen Schmelzpunktes. Der Druck nach dem Entspannen kann in einem großen Bereich schwanken. Für den Fall, daß ein Tempern unmittelbar angeschlossen wird, wird auf höheren Druck entspannt, im anderen Fall ist ein Entspannen bis auf Atmosphärendruck möglich. Der Druck nach dem Entspannen liegt demnach bevorzugt bei etwa 1 bis 150 oder 100 bar, besonders bevorzugt bei etwa 1 bis 60 bar. Er kann aber auch bei etwa 10 bis 20 bar liegen. Für den Fall, daß die Temperatur beim Entspannen und Verfestigen gleich bleibt oder ansteigt, ist es vorteilhaft, das sich dabei aus der Melaminschmelze bildende feste Melamin etwa bei jenen Temperatur- und Druckbedingungen verweilen zu lassen (tempern), bei denen es sich nach dem Entspannen abscheidet.

Mit Hilfe des erfindungsgemäßen Verfahrens kann Melamin mit einer Reinheit von über 99 Gew. % erhalten werden. Je nach den gewählten Temperatur- und Druckbedingungen vor und nach dem Entspannen kann Melamin auch mit einer Reinheit von bis zu 99,9 Gew.%, teilweise über 99,99 Gew.% erhalten werden, wobei insbesondere hohe Ammoniakdrücke sowie Temperaturen nahe dem Schmelzpunkt des Melamins von Vorteil sind.

Das erfindungsgemäße Verfahren kann sowohl diskontinuierlich als auch kontinuierlich durchgeführt werden. Das Verfahren eignet sich bevorzugt im Anschluß an einen Prozeß zur Herstellung von Melamin, insbesondere im Anschluß an einen beliebigen Hochdruckprozeß zur Herstellung von Melamin aus Harnstoff, bei dem das Melamin zunächst in flüssiger Form als Schmelze anfällt. In Melamin-Hochdruckprozessen wird Melamin üblicherweise bei Drücken von etwa 70 bis 800 bar und bei Temperaturen ― abhängig vom gewählten Druck - von mindestens etwa 360 °C in flüssiger Form als Schmelze erhalten. Die bei der Melaminsynthese entstehenden, insbesondere NH₃, CO₂ und gasförmiges Melamin enthaltenden Offgase werden üblicherweise durch Hindurchleiten durch eine Harnstoffschmelze gewaschen. Dabei wird die Harnstoffschmelze durch die heißen Offgase erwärmt und vorteilhafterweise zur Melaminsynthese in einen Melaminreaktor geführt, während die gereinigten Offgase vorteilhafterweise in einen Harnstoffreaktor geführt werden. Die Offgase können entweder direkt in den Harnstoffreaktor geführt werden, oder sie werden beispielsweise mit Hilfe von Ammoncarbonat- oder Ammoncarbamatlösungen, die beispielsweise in der Melaminanlage oder der Harnstoffanlage anfallen, kondensiert. Die dabei anfallende Wärme kann beispielsweise zum Vorwärme des in der Harnstoffanlage eingesetzten Ammoniaks oder zur Produktion von Dampf verwendet werden.
Nach Abtrennen der Offgase kann die Melaminschmelze vorteilhafterweise gestrippt werden, beispielsweise mit NH₃, wodurch vor allem restliches CO₂ entfernt wird. Weiters ist es vorteilhaft, die Melaminschmelze in einem Aging-Behälter verweilen zu lassen. Es ist jedoch auch möglich, die aus dem Reaktor kommende Melaminschmelze nach Abtrennen der Offgase direkt mit überschüssigem Ammoniak zu vermischen.

Der Vorteil des erfindungsgemäßen Verfahrens liegt vor allem darin, daß unabhängig von der vom jeweiligen Druck und der jeweiligen Temperatur abhängigen Sättigung der Melaminschmelze mit gelöstem Ammoniak, auch darüber hinaus der Melaminschmelze mehr oder weniger Ammoniak zugeführt werden kann. Dadurch wird es möglich, die Temperatur bei der Entspannung der Melaminschmelze, entsprechend den verfahrensbedingten und produktbedingten Erfordernissen und in Abhängigkeit von der Menge des überschüssigen Ammoniaks in der Schmelze, einfach und in einem breiten Bereich zu regeln. Damit ist beispielsweise auch die Möglichkeit geschaffen, festes Melamin in guter Qualität auch bei niederen Drücken herzustellen. Ein weiterer Vorteil liegt auch in der Flexibilität, Melamin in der für die jeweiligen Anwendungen geforderten Reinheit herstellen zu können. Für den Fall, daß bei der Verfestigung keine Abkühlung erfolgt, ergibt sich der zusätzliche Vorteil, daß für ein eventuelles Tempern keine bzw. nur geringe zusätzliche Wärme zugeführt werden muß.

## Patentansprüche

1. Verfahren zur Herstellung von festem Melamin durch Entspannen von flüssigem, ammoniakhältigem Melamin, **dadurch gekennzeichnet, dass**
a) das flüssige ammoniakhältige Melamin mit überschüssigem Ammoniak vermischt wird, wobei sich eine Dispersion von gasförmigen Ammoniak und flüssigem Melamin bildet,
b) die Dispersion gegebenenfalls unter Ammoniakdruck verweilen gelassen wird,
c) die Dispersion entspannt wird, wobei sich Melamin in fester Form abscheidet,
d) das feste Melamin gegebenenfalls unter Ammoniakdruck verweilen gelassen wird,
e) worauf in beliebiger Reihenfolge gegebenenfalls auf Atmosphärendruck weiter entspannt, auf Raumtemperatur abgekühlt und das Melamin isoliert wird.

2. Verfahren gemäß Anspruch 1, **dadurch gekennzeichnet, dass** das flüssige, ammoniakhältige Melamin mit überschüssigem Ammoniak vermischt und gleichzeitig entspannt wird, wobei sich Melamin in fester Form abscheidet.

3. Verfahren gemäß Anspruch 2, **dadurch gekennzeichnet, dass** das Mischen des flüssigen ammoniakhältigen Melamins mit überschüssigem Ammoniak unter gleichzeitiger Entspannung mit Hilfe von Injektoren oder Ejektoren erfolgt.

4. Verfahren gemäß einem der Ansprüche 1 bis 3, **dadurch gekennzeichnet, dass** sich Melamin beim Entspannen bei gleicher Temperatur oder unter Erhöhung der Temperatur in fester Form abscheidet.

5. Verfahren gemäß einem der Ansprüche 1 bis 4, **dadurch gekennzeichnet, dass** das flüssige ammoniakhältige Melamin mit Ammoniak gesättigt ist.

6. Verfahren gemäß einem der Ansprüche 1 bis 5, **dadurch gekennzeichnet, dass** das flüssige ammoniakhältige Melamin bei Drücken von etwa 60 bar bis 600 bar mit überschüssigem Ammoniak vermischt und anschließend auf einen Druck von etwa 1 bis 60 bar entspannt wird, wobei sich Melamin in fester Form abscheidet.

7. Verfahren gemäß Anspruch 6, **dadurch gekennzeichnet, dass** das flüssige ammoniakhältige Melamin bei Drücken von etwa 80 bis 350 bar mit überschüssigem Ammoniak vermischt wird.

8. Verfahren gemäß Anspruch 6 oder 7, **dadurch gekennzeichnet, dass** das flüssoge ammoniakhältige Melamin, das mit überschüssigem Ammoniak vermischt wird, anschließend auf einen Druck von etwa 1 bis 20 bar entspannt wird, wobei sich Melamin in fester Form abscheidet.

9. Verfahren gemäß einem der Ansprüche 1 bis 8, **dadurch gekennzeichnet, dass** das flüssige ammoniakhältige Melamin in einem Temperaturbereich von etwa 60 °C über dem vom jeweiligen Ammoniakdruck abhängigen Schmelzpunkt des Melamins bis knapp über dem vom jeweiligen Ammoniakdruck abhängigen Schmelzpunkt des Melamins mit überschüssigem Ammoniak vermischt wird.

10. Verfahren nach Anspruch 9, **dadurch gekennzeichnet, dass** das flüssige ammoniakhältige Melamin bei Temperaturen, die bis 1 und etwa 20 °C über dem vom jeweiligen Ammoniakdruck abhängigen Schmelzpunkt des Melamins liegen, mit überschüssigem Ammoniak vermischt wird.

11. Verfahren gemäß einem der Ansprüche 1 bis 10, **dadurch gekennzeichnet, dass** es an einen kontinuierlichen Hochdruckprozeß zur Herstellung von Melamin aus Harnstoff anschlossen wird.

12. Verfahren gemäß einem der Ansprüche 1 bis 11, **dadurch gekennzeichnet, dass** die Entspannung in einen separaten, eine Ammoniakatmosphäre enthaltenden Behälter erfolgt.

13. Verfahren gemäß einem der Ansprüche 1 bis 12, **dadurch gekennzeichnet, dass** während der Entspannung zusätzlich Ammoniak zugeführt wird.

14. Verfahren gemäß einem der Ansprüche 1 bis 13, **dadurch gekennzeichnet, dass** das flüssige ammoniakhältige Melamin vor dem Entspannen unter Ammoniakdruck verweilen gelassen wird.

15. Verfahren gemäß Anspruch 14, **dadurch gekennzeichnet, dass** das flüssige ammoniakhältige Melamin vor dem Entspannen unter Ammoniakdruck während etwa 1 min bis 10 Stunden verweilen gelassen wird.

16. Verfahren gemäß Anspruch 14 oder 15, **dadurch gekennzeichnet, dass** das Verweilen bei einer Temperatur, die etwa 1 bis 20 °C über dem vom jeweiligen Ammoniakdruck abhängigen Schmelzpunkt des Melamins liegt erfolgt.

17. Verfahren gemäß einem der Ansprüche 1 bis 16, **dadurch gekennzeichnet, dass** die Dispersion von Ammoniak und flüssigem Melamin bei einer Temperatur, die etwa 1 bis 20 °C über dem vom jeweiligen Ammoniakdruck abhängigen Schmelzpunkt des Melamins liegt, entspannt wird.

18. Verfahren gemäß einem der Ansprüche 1 bis 17, **dadurch gekennzeichnet, dass** das feste Melamin nach dem Entspannen während etwa 1 min bis 2 h in einem Temperaturbereich, der zwischen etwa 150 °C und dem vom jeweiligen Ammoniakdruck abhängigen Schmelzpunkt des Melamins liegt, in einer Ammoniakatmosphäre verweilen gelassen wird.

19. Verfahren gemäß einem der Ansprüche 1 bis 18, **dadurch gekennzeichnet, dass** das Mischen des flüssigen ammoniakhältigen Melamins mit überschüssigem Ammoniak unter Bildung einer Dispersion mit Hilfe von Rührern, Statikmischem, Injektoren oder Ejektoren erfolgt.

## Claims

1. Process for preparing solid melamine by depressurizing liquid, ammonia-containing melamine, **characterized in that**
a) the liquid ammonia-containing melamine is mixed with excess ammonia, whereupon a dispersion of gaseous ammonia and liquid melamine forms,
b) where appropriate, the dispersion is aged under the pressure generated by ammonia,
c) the dispersion is depressurized, whereupon solid melamine precipitates,
d) where appropriate, the solid melamine is aged under the pressure generated by ammonia,
e) and then, where appropriate and in any desired sequence, there is further depressurization to atmospheric pressure, and cooling to room temperature, and the melamine is isolated.

2. Process according to Claim 1, **characterized in that** the liquid, ammonia-containing melamine is mixed with excess ammonia and simultaneously depressurized, whereupon solid melamine precipitates.

3. Process according to Claim 2, **characterized in that** the mixing of the liquid ammonia-containing melamine with excess ammonia takes place with simultaneous depressurization with the aid of injectors or of ejectors.

4. Process according to any of Claims 1 to 3, **characterized in that** solid melamine precipitates during depressurization at the same temperature, or with an increase in the temperature.

5. Process according to any of Claims 1 to 4, **characterized in that** the liquid ammonia-containing melamine has been saturated with ammonia.

6. Process according to any of Claims 1 to 5, **characterized in that** the pressures at which the liquid ammonia-containing melamine is mixed with excess ammonia are from about 60 to 600 bar, and that the liquid ammonia-containing melamine is then depressurized to a pressure of from about 1 to 60 bar, whereupon solid melamine precipitates.

7. Process according to Claim 6, **characterized in that** the pressures at which the liquid ammonia-containing melamine is mixed with excess ammonia are from about 80 bar to 350 bar.

8. Process according to Claim 6 or 7, **characterized in that** the liquid ammonia-containing melamine which is mixed with excess ammonia is then depressurized to a pressure of from about 1 bar to 20 bar, whereupon solid melamine precipitates.

9. Process according to any of Claims 1 to 8, **characterized in that** the temperature range within which the liquid ammonia-containing melamine is mixed with excess ammonia is from about 60°C above the melting point of the melamine, which depends on the ammonia pressure used, to just above the melting point of the melamine, which depends on the ammonia pressure used.

10. Process according to Claim 9, **characterized in that** the temperatures at which the liquid ammonia-containing melamine is mixed with excess ammonia are from 1 to about 20°C above the melting point of the melamine, which depends on the ammonia pressure used.

11. Process according to any of Claims 1 to 10, **characterized in that** it is downstream of a continuous high-pressure process for preparing melamine from urea.

12. Process according to any of Claims 1 to 11, **characterized in that** the depressurization takes place in a separate container in which an atmosphere of ammonia is present.

13. Process according to any of Claims 1 to 12, **characterized in that** additional ammonia is introduced during the depressurization.

14. Process according to any of Claims 1 to 13, **characterized in that** the liquid ammonia-containing melamine is aged under the pressure generated by ammonia prior to the depressurization.

15. Process according to Claim 14, **characterized in that** the liquid ammonia-containing melamine is aged for from about 1 min to 10 hours under the pressure generated by ammonia prior to the depressurization.

16. Process according to Claim 14 or 15, **characterized in that** temperature at which the aging takes place is from about 1 to 20°C above the melting point of the melamine, which depends on the ammonia pressure used.

17. Process according to any of Claims 1 to 16, **characterized in that** the dispersion of ammonia and liquid melamine is depressurized at a temperature which is from about 1 to 20°C above the melting point of the melamine, which depends on the ammonia pressure used.

18. Process according to any of Claims 1 to 17, **characterized in that**, after the depressurization, the solid melamine is aged in an atmosphere of ammonia for from about 1 min to 2 h, in a temperature range from about 150°C to the melting point of the melamine, which depends on the ammonia pressure used.

19. Process according to any of Claims 1 to 18, **characterized in that** the mixing of the liquid ammonia-containing melamine with excess ammonia, forming a dispersion, takes place with the aid of stirrers, static mixers, injectors or ejectors.

## Revendications

1. Procédé de fabrication de mélamine solide par détente de mélamine fluide, contenant de l'ammoniac, **caractérisé en ce que**
a) la mélamine fluide contenant de l'ammoniac est mélangée à de l'ammoniac en excès, une dispersion d'ammoniac gazeux et de mélamine fluide se formant,
b) la dispersion est laissée au repos, le cas échéant, sous pression d'ammoniac,
c) la dispersion est détendue, la mélamine se déposant sous forme solide,
d) la mélamine solide est laissée au repos, le cas échéant, sous pression d'ammoniac,
e) à la suite de quoi, dans une séquence quelconque, le cas échéant, sous pression atmosphérique, on poursuit la détente, on refroidit à la température ambiante et on isole la mélamine.

2. Procédé selon la revendication 1, **caractérisé en ce que** la mélamine fluide, contenant de l'ammoniac est mélangée à de l'ammoniac en excès et subit simultanément une détente, la mélamine se déposant sous forme solide.

3. Procédé selon la revendication 2, **caractérisé en ce que** le mélange de la mélamine fluide, contenant de l'ammoniac, se fait sous détente simultanée à l'aide d'injecteurs ou d'éjecteurs avec de l'ammoniac en excès.

4. Procédé selon l'une quelconque des revendications 1 à 3, **caractérisé en ce que** la mélamine, lors de la détente, se dépose sous forme solide à la même température ou avec une augmentation de la température.

5. Procédé selon l'une quelconque des revendications 1 à 4, **caractérisé en ce que** la mélamine fluide, contenant de l'ammoniac est saturée d'ammoniac.

6. Procédé selon l'une quelconque des revendications 1 à 5, **caractérisé en ce que** la mélamine fluide, contenant de l'ammoniac, est mélangée à de l'ammoniac en excès à des pressions d'environ 60 bars à 600 bars et est ensuite détendue à une pression d'environ 1 à 60 bars, la mélamine se déposant sous forme solide.

7. Procédé selon la revendication 6, **caractérisé en ce que** la mélamine fluide, contenant de l'ammoniac, est mélangée à de l'ammoniac en excès à des pressions d'environ 80 à 350 bars.

8. Procédé selon la revendication 6 ou 7, **caractérisé en ce que** la mélamine fluide, contenant de l'ammoniac, est mélangée à de l'ammoniac en excès, est ensuite détendue à une pression d'environ 1 à 20 bars, la mélamine se déposant sous forme solide.

9. Procédé selon l'une quelconque des revendications 1 à 8, **caractérisé en ce que** la mélamine fluide, contenant de l'ammoniac, est mélangée à de l'ammoniac en excès dans un domaine de températures allant d'environ 60°C au-dessus du point de fusion de la mélamine, dépendant à chaque fois de la pression d'ammoniac jusqu'à tout juste au-dessus du point de fusion de la mélamine, dépendant à chaque fois de la pression d'ammoniac.

10. Procédé selon la revendication 9, **caractérisé en ce que** la mélamine fluide, contenant de l'ammoniac est mélangée à de l'ammoniac en excès à des températures qui se situent de 1 à environ 20 °C au-dessus du point de fusion de la mélamine, dépendant à chaque fois de la pression d'ammoniac.

11. Procédé selon l'une quelconque des revendications 1 à 10, **caractérisé en ce qu'**il est raccordé à un processus haute pression en continu, en vue de la fabrication de mélamine à partir d'urée.

12. Procédé selon l'une quelconque des revendications 1 à 11, **caractérisé en ce que** la détente se fait dans un récipient séparé, contenant une atmosphère d'ammoniac.

13. Procédé selon l'une quelconque des revendications 1 à 12, **caractérisé en ce que** de l'ammoniac est acheminé en sus lors de la détente.

14. Procédé selon l'une quelconque des revendications 1 à 13, **caractérisé en ce que** la mélamine fluide, contenant de l'ammoniac est laissée, avant la détente, au repos sous pression d'ammoniac.

15. Procédé selon la revendication 14, **caractérisé en ce que** la mélamine fluide, contenant de l'ammoniac est laissée, avant la détente, au repos sous pression d'ammoniac pendant une période d'environ 1 minute à 10 heures.

16. Procédé selon la revendication 14 ou 15, **caractérisé en ce que** la mise au repos se fait à une température, qui se situe à environ de 1 à 20°C au-dessus du point de fusion de la mélamine, dépendant à chaque fois de la pression d'ammoniac.

17. Procédé selon l'une quelconque des revendications 1 à 16, **caractérisé en ce que** la dispersion d'ammoniac et de mélamine fluide est détendue à une température, qui se situe à environ de 1 à 20°C au-dessus du point de fusion de la mélamine, dépendant à chaque fois de la pression d'ammoniac.

18. Procédé selon l'une quelconque des revendications 1 bis 17, **caractérisé en ce que** la mélamine solide, après la détente, est laissée au repos dans une atmosphère d'ammoniac pendant une période d'environ 1 minute à 2 heures dans un domaine de températures qui se situe entre environ 150°C et le point de fusion de la mélamine, dépendant à chaque fois de la pression d'ammoniac.

19. Procédé selon l'une quelconque des revendications 1 à 18, **caractérisé en ce que** le mélange de la mélamine fluide, contenant de l'ammoniac, à de l'ammoniac en excès, se fait par formation d'une dispersion, à l'aide de tubes, de mélangeurs statiques, d'injecteurs ou d'éjecteurs.
